# EUROPEAN PATENT APPLICATION

(11) **EP 1 987 787 A1**
(43) Date of publication of application: **05.11.2008**
(21) Application number: 08162662.4
(22) Date of filing: 15.02.2005
(51) Int. Cl.: A61B 17/22, A61M 25/00, A61F 2/01

(54) **Embolectomy capture sheath**

(30) Priority: 19.02.2004 US 545801
(62) Divisional of application: 05713655.8
(71) Applicant: APPLIED MEDICAL RESOURCES CORPORATION, Rancho Santa Margarita, CA 92688 (US)
(72) Inventor: Nguyen, Eric, Corona, SC 92879 (US); Hart, Charles C., Summerville, SC 29483-8949 (US)
(74) Representative: Fitchett, Stuart Paul

(57) **Abstract**

An access device adapted for use in a body conduit is provided comprising an outer tube having a proximal end and a distal end, an inner tube disposed coaxially with the outer tube having a proximal end and a distal end and an expandable portion having a first end coupled to the distal end of the outer tube and a second end coupled to the distal end of the inner tube. The outer tube and the inner tube are movable relative to each other to transform the expandable portion between a low-profile state and a high-profile state and at least one of the outer and inner tubes comprises a wire-reinforced tube or is formed from a plurality of individual, discrete generally ring-shaped elements arranged in series and fused or bonded together to form a continuous tubular structure. The access device may further comprise an actuator coupled to one of the outer and inner tubes and being movable relative to the other of the outer and inner tubes to transform the expandable portion between the low- and high-profile states. The expandable portion may be formed with a braid material and has the shape of a cone. The ring-shaped elements may be formed of a thermoplastic or a thermoset material, and they may include at least one of plastic rings, metallic rings, un-reinforced plastic rings and metal reinforced plastic rings assembled along the length of at least one of the tubes to provide variable flexibility and kink-resistance. The wire-reinforced tube is formed by coating a wire with a plastic material, wrapping the coated wire around a mandrel forming a plurality of windings, and heating the wound coated wire until the plastic material melts and bonds the windings forming the wire-reinforced tube.

## Description

This is a non-provisional application claiming the priority of provisional application Serial No. 60/545,801, filed on February 19, 2004, entitled "Embolectomy Capture Sheath," which is fully incorporated herein by reference.

### BACKGROUND OF THE INVENTION

### Field of the Invention

This invention generally relates to devices for removing obstructing materials from body passages and, more particularly, to an access device configurable between a first small diameter for initial insertion into a body passage and a second larger diameter for effecting the removal of an obstructing material.

### Discussion of Related Art

U.S. Patent Nos. 5,971,938, 5,846,251, and 6,210,370 to Applicants relate to the present technology and are all herein incorporated by reference.

There are many devices for removing obstructing materials from a body passage such as a blood vessel. The obstructing materials may include plaque, thrombus, embolus, clots and fatty deposits. In other cases, the obstructions may result from stones and strictures. Catheters are commonly inserted into vessels for the purpose of dislodging obstructing materials from the vessel walls. In a prevalent technique commonly referred to as an embolectomy/thrombectomy procedure, a balloon tipped catheter is introduced through a surgical incision a nd into a blood vessel. The balloon tipped catheter is advanced to the location of the obstructing material or occlusion, and the balloon is then inflated at a point within the vessel beyond the point of the obstructing material. The catheter including the attached balloon is then pulled back to the point of insertion. In this manner, the balloon pulls the obstructing material to the point of insertion where it is removed through the incision. When the obstructing material is detached from a vessel wall using this technique, the obstructing material will often have a tendency to disperse and migrate with the flow of blood within the vessel. This dispersion can make collection and removal of the obstructing material difficult, and the migration can put the patient at risk of acute trauma. A drawback associated with this embolectomy technique involves the efficient collection and removal of obstructing material while preventing migration and dispersion of the obstructing material.

Other percutaneous procedures exist in the prior art for recanalization of vessels. One percutaneous procedure involves the use of laser energy to vaporize the stenotic material. Another percutaneous procedure, commonly referred to as aspiration embolectomy/thrombectomy, relies on a negative pressure to collect the obstructing material.

In the case of a balloon catheter, for example, percutaneous or minimally invasive access to a blood vessel requires the catheter to have a very small diameter to fit through a corresponding small incision in the blood vessel. Once the catheter is in the blood vessel, however, portions of the catheter need to assume a large profile removal interface for efficient removal of the obstructing material from the lumen of the blood vessel. In an attempt to meet this dual functional requirement of small incision diameter and large intraluminal operating diameter, the devices of the prior art have incorporated sheaths in conjunction with catheters. U.S. Patent No. 5,011,488 to Ginsburg discloses the use of an expanding funnel-shaped sheath for use in withdrawing thrombus or embolus (obstructing material) from a blood vessel. The funnel is deployed by extending the expanding funnel-shaped sheath from within a second sheath to thereby allow the compressed funnel to expand radially. Use of this second sheath tends to increase the overall diameter of the device, thus increasing the size of the incision required for insertion of the device. In addition to not achieving an optimally small insertion diameter, this device is also unable to obtain an optimally large intraluminal operating diameter. An optimally large intraluminal operating diameter would allow for better insertion and removal of larger instruments through the sheath. This prior art technique of inserting both the introducer sheath and the pre-shaped funnel sheath into the body passage, and of subsequently removing the introducer sheath, requires a large introduction incision to form a seal around a smaller diameter pre-shaped funnel sheath after the introducer sheath is removed. In other words, the double sheath combination of the prior art requires an initial incision into the body passage large enough to accommodate the introducer sheath and, subsequently, small enough to adequately form a seal around the smaller pre-formed funnel sheath left in place after the introducer sheath is removed. Since the initial incision cannot be subsequently reduced in size to accommodate the funnel sheath, a good seal in this prior art system is difficult to obtain.

Other devices, which provide access to relatively inaccessible regions of blood vessels, are disclosed in U.S. Patent No. 4,530,698 and U.S. Patent No. 4,437,859. Needle and sheath combinations for use in drug delivery, blood withdrawal, and dialysis have been proposed by the prior art, but these devices incorporate different structure to solve a problem, which is different than the removal of obstructing material from a body passage. U.S. Patent No. 5,234,425 to Fogarty discloses a variable diameter sheath constructed of a composite elastomeric material that may be stretched to reduce the diameter. This variable diameter sheath, however, is not used for the removal of obstructing material. Instead, the primary goal of this device is to provide a lining of a body passage with a thin walled single thickness interior sheath, which is introduced into the body passage in a reduced diameter condition and subsequently expanded to snugly fit the interior wall of the body passage. The variable diameter sheath incorporates a tubular braid encapsulated within a coating of high elongation silicone polymer. Having stated the above, there remains a need in the art for an improved access device sized and configured to facilitate removal of obstructing materials from a body passage, the access device being formed from thin walled reinforced tubing.

### SUMMARY OF THE INVENTION

According to one aspect of the invention, an access device is provided comprising an outer tube having a proximal end and a distal end, an inner tube disposed coaxially with the outer tube having a proximal end and a distal end, and an expandable portion having a first end coupled to the distal end of the outer tube and a second end coupled to the distal end of the inner tube. With this aspect, the outer tube and the inner tube are movable relative to each other to transform the expandable portion between a low-profile state and a high-profile state, and at least one of the outer tube and the inner tube is formed from a plurality of individual, discrete, generally ring-shaped elements arranged in series and fused or bonded together to form a continuous tubular structure. The access device may further comprise an actuator coupled to one of the outer and inner tubes and being movable relative to the other of the outer and inner tubes to transform the expandable portion between the low-profile state and the high-profile state. The expandable portion may be formed with a braid material and, in the high-profile state, has the shape of a cone or funnel.

The ring-shaped elements may be formed of a thermoplastic or a thermoset material, and they may include at least one of plastic rings, metallic rings, un-reinforced plastic rings and metal reinforced plastic rings assembled along the length of at least one of the outer and inner tubes to provide variable flexibility and kink-resistance. It is appreciated that the ring-shaped elements may have different flexural modulus, and that the ring-shaped elements may include a combination of flexible and rigid ring-shaped elements assembled along different portions or sections of one of the outer and inner tubes. In another aspect, the ring-shaped elements are metallic and are bonded with a resilient, flexible elastomeric adhesive. The ring-shaped elements may also have different lengths and may be fused closer or further apart to one another depending on the characteristics of a portion or section of one of the outer and inner tubes. The outer tube may be constructed from braid-reinforced element, Polyimide, rubber, polyvinyl chloride, polyurethane or polyester.

In another aspect of the invention, an access device is provided comprising an outer tube having a proximal end and a distal end, an inner tube disposed coaxially with the outer tube having a proximal end and a distal end, and an expandable portion having a first end coupled to the distal end of the outer tube and a second end coupled to the distal end of the inner tube. With this aspect, the outer tube and the inner tube , are movable relative to each other to transform the expandable portion between a low-profile state and a high-profile state, and at least one of the outer tube and the inner tube comprises a wire-reinforced tube. The wire-reinforced tube is manufactured by the process comprising the steps of coating a wire with a plastic material, wrapping the coated wire around a mandrel forming a plurality of windings, and heating the wound coated wire until the plastic material melts and bonds the windings forming the wire-reinforced tube. The plastic material may comprise at least one of polyurethane, a thermoplastic material and a thermoset material. The wire may comprise at least one of a metallic material and a second plastic material. The wire may be coated with the plastic material in a coextrusion process. The wire-reinforced tube may have a wall thickness of about 0.015" or less, or it may have an inner diameter ranging from about 0.026" to about 0.75", with the tolerance on the inner diameter being on the order of 0.001" or less. The process of manufacturing the wire-reinforced tube may further comprise each of the steps of compressing the windings as the coated wire is being heated, providing a mold to compress the windings, and/or removing the wire-reinforced tube from the mandrel after the tube is cooled.

In yet another aspect of the invention, an access device is provided comprising an outer tube having a proximal tube end, a distal tube end, and a lumen extending between the proximal tube end and the distal tube end; an obturator assembly having a proximal obturator end a distal obturator end, and being removably and concentrically disposed within the lumen of the outer lube; an expandable portion having a proximal end being connected to the distal tube end and a distal end being detachably connected to the distal obturator end, the expandable portion being expandable by relative movement between the expandable portion proximal end and the expandable portion distal end, resulting from relative movement of the outer tube and the obturator assembly in opposite directions; and the outer tube comprises a wire-reinforced tube or is formed from a plurality of individual, discrete, generally ring-shaped elements arranged in series and fused or bonded together forming a continuous tubular structure.

The invention, together with additional features and advantages thereof, may best be understood by reference to the following description taken in connection with the accompanying illustrative drawings.

### DESCRIPTION OF THE DRAWINGS

FIG. 1 illustrates an access device in accordance with an embodiment of the invention to provide access to a body conduit;
FIGS. 2(a) - 2(c) illustrate side sectional views of the access device of the invention in a first low-profile condition, a second high-profile condition, and a third low-profile condition, respectively;
FIG. 3 illustrates a side sectional view of the access device of the invention having a flexible inner tube made of thin-walled wire-reinforced tubing;
FIG. 4 illustrates the expandable portion of the access device of the invention in the first low-profile condition for insertion into a body conduit;
FIG. 5 illustrates the expandable portion of the access device of the invention in the second high-profile condition for use within a body conduit;
FIG. 6 illustrates the expandable portion of the access device of the invention in the third low-profile condition for withdrawal from a body conduit;
FIG. 7 illustrates the access device of the invention inserted into a body conduit in the first condition;
FIG. 8 illustrates the access device of the invention inserted into a body conduit in the second condition;
FIG. 9 illustrates the access device of the invention inserted into a body conduit in the third condition;
FIG. 10 illustrates a side sectional view of an access sheath assembly according to another aspect of the invention;
FIG. 11 illustrates a side sectional view of the access sheath of FIG. 10 with the obturator having an expandable cone deployed;
FIG. 12 illustrates a side sectional view of the access sheath of FIG. 10 with the expandable containment member of the outer flexible tube deployed;
FIGS. 13(a) -13(c) illustrate a therapeutic balloon catheter for use with the outer tube of an access device of the invention to facilitate removal of an obstructing material;
FIGS. 14(a) -14(d) illustrate a cowling for covering the expandable cone of an access device of the invention; and
FIGS. 15(a) and 15(b) illustrate side sectional views of access devices having at least one of the outer and inner tubes formed with a braid element or that is braid-reinforced.

### DESCRIPTION OF THE INVENTION

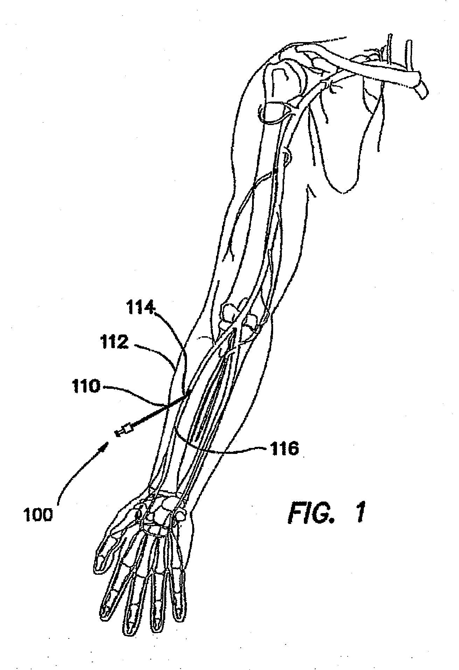

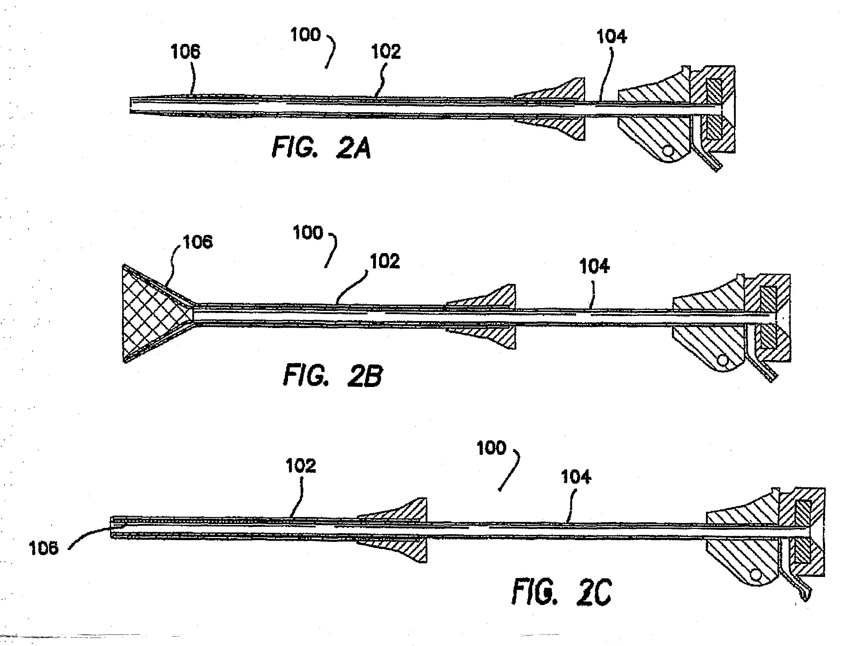

Referring to FIG. 1, there is shown an access device 100 of the invention being placed through a puncture site 110 in the skin 112 of a patient, and through a vessel puncture 114 of a body passage 116. FIGS. 2(a) - 2(c) illustrate side sectional views of the access device 100 of the invention having a first outer flexible tube 102, a second inner, coaxial, flexible tube 104, and an expandable portion 106 disposed at a distal portion of the first and second tubes 102, 104. The expandable portion 106 may be taken from a first low-profile condition (FIG. 2(a)) to a second high-profile condition (FIG. 2(b)) and subsequently to a third low-profile condition (FIG. 2(c)) by the relative axial movements of the first and second tubes 102, 104 as further described below. The first low-profile condition is sized and configured to facilitate entry into a body conduit such as a vein or an artery. The second high-profile condition is sized and configured to facilitate the introduction of foreign material into the inner tube 104 of the access device 100. The second high-profile condition can be configured and shaped like a cone. The third low-profile condition places the expandable portion 106 within the outer tube 102 of the access device 100 to facilitate removal of the access device 100 from a body conduit.

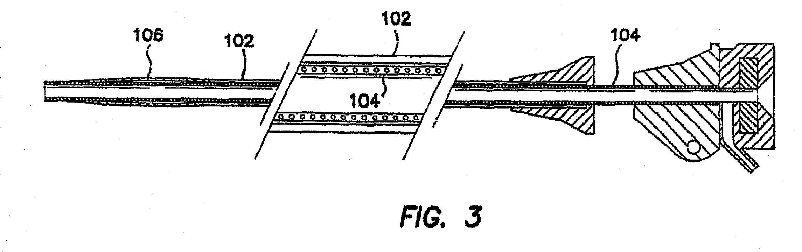

In one aspect of the invention, FIG. 3 illustrates the inner flexible tube 104 as being constructed from thin walled wire-reinforced tubing as described in Applicants' copending patent application, "Medical Tubing Having Variable Characteristics and Method for Making Same," Serial No. 10/766,138, filed January 28, 2004, which is fully incorporated herein by reference, and the outer flexible tube 102 is constructed from a thin walled material such as Polyimide or any of a wide variety of biologically compatible materials such as rubber, polyvinylchloride, polyurethane, polyester and the like. It is appreciated that the outer and inner tubes 102, 104 may be formed from the same or different material. The expandable portion 106 is typically formed with a braid material. In some instances, however, it may be preferable to have the expandable portion 106 not be impervious to fluid. In the case of uncoated braid, this serves the purpose of allowing fluid to be squeezed through the cone while still capturing the solid embolic material.

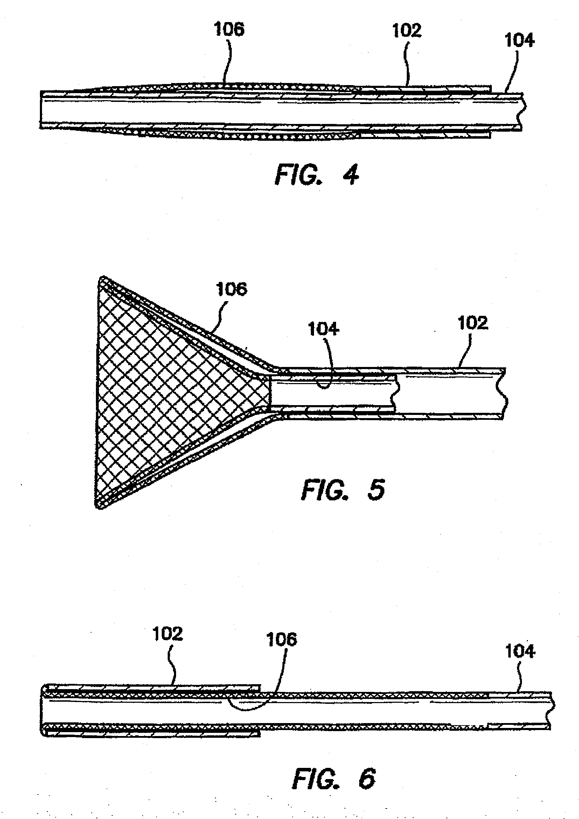

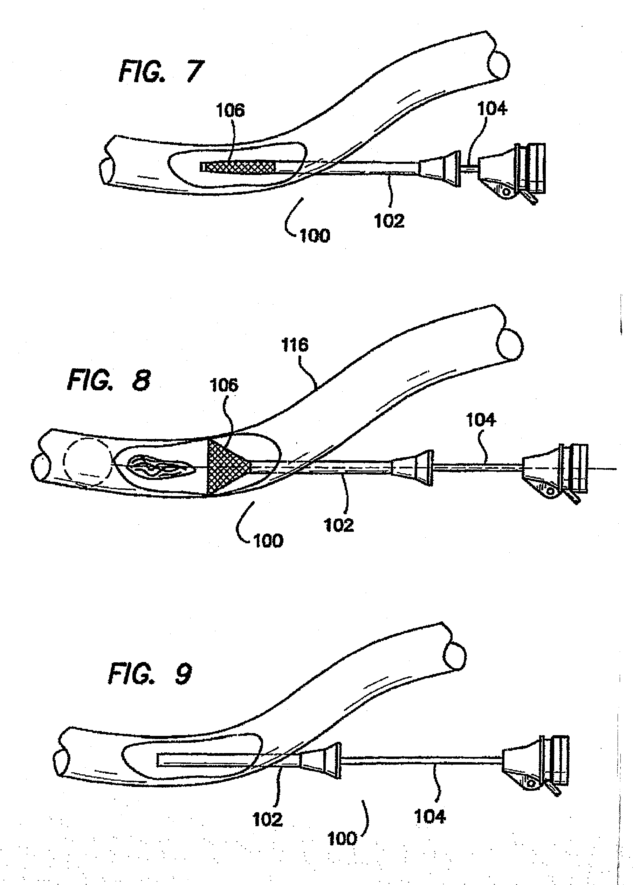

The length of the tube will vary depending on the intended use. The inner tube 104 is disposed within a lumen of outer tube 102. The outer diameter of the inner tube 104 is slightly less than the diameter of the lumen of the outer tube 102 in order to provide the necessary clearance. Accordingly, the outer tube 102 operates to slide over the inner tube 104 and in so doing allows the expandable portion 106 to expand to the diameter of the graft or vessel. This allows substantially all of the embolic material to be captured in the expandable portion 106 and subsequently withdrawn and/or aspirated from the graft or vessel. It is appreciated that the outer tube 102 can slide coaxially over the inner tube 104 and place the expandable portion 106 in a compressed condition when it is advanced beyond the distal end of the inner tube 104. FIGS. 4 and 7 illustrate the expandable portion 106 in the first low-profile condition for insertion into a body conduit; FIGS. 5 and 8 illustrate the expandable portion 106 in the second high-profile condition for use within the body conduit to capture an obstructing material; and FIGS. 6 and 9 illustrate the expandable portion 1 06 in the third low-profile condition for withdrawal from the body conduit.

As illustrated in FIGS. 4 and 6, the expandable portion 106 may be attached to both the distal ends of the inner tube 104 and the outer tube 102. With this configuration, when the outer tube 102 is retracted proximally to the limit allowed by the expandable portion 106, the expandable portion 106 will form a coaxial sheath around the inner tube 104. Conversely, if the inner tube 104 is retracted proximally to the outer tube 102, the expandable portion 106 material will form an inner sheath at the limit of the movement.

As stated above, the inner tube 104 may be formed from a wire-reinforced material. Advantages of wire-reinforced tubing include, among other things, thin wall, flexibility and kink resistance. It is appreciated that both the inner tube 104 and the outer tube 102 may be constructed of wire-reinforced material. That is, the inner and outer tubes 104, 102 can be formed with a thermoset material in a process of the invention as follows. A spring wire is wound on a mandrel with the desired pitch. It is then coated in the thermoset such as silicone. Next, the silicone compression tube is placed over the spring wire and the assembly is allowed to cure. The tube is then removed from the silicone tube and mandrel. Springs can be pre-wound and can be made of materials other than steel that would otherwise not tolerate the heat required to flow a thermoplastic.

In another aspect, each of the inner tube 104 and the outer tube 102 may be constructed of a plurality of individual, discrete, generally ring-shaped elements arranged in series to form a continuous tubular structure. The ring-shaped elements may be formed of a thermoplastic or a thermoset material. The ring-shaped elements may be arranged in series and subsequently fused or bonded by heat or chemical reaction to form a substantially continuous form.

It is appreciated that tubing constructed from a series of individual, discreet elements may be bent, shaped or coiled without kinking. In particular, the tubing of the outer and inner tubes 102, 104 may have variable characteristics along the length. This may be achieved, for example, by the use of the ring-shaped elements to provide different flexural modulus. For instance, a length of tubing may be constructed such that flexible ring-shaped elements are separated by rigid ring-shaped elements, i.e., a flexible portion of a tubular structure may be formed adjacent to a rigid or semi-rigid portion of the tubular structure to provide variable flexibility. Such construction allows softer, more flexible material to be displaced and stretched along a curvature so that the rigid material is not deformed. One embodiment may comprise a thermoplastic of a very rigid nature spaced by a compatible thermoplastic of a very soft nature. In other words, the ring-shaped elements may be formed of two or more different materials having different chemical composition and hardness that are alternately fused or bonded together to form a continuous tube having circumferential portions that are alternately rigid and flexible.

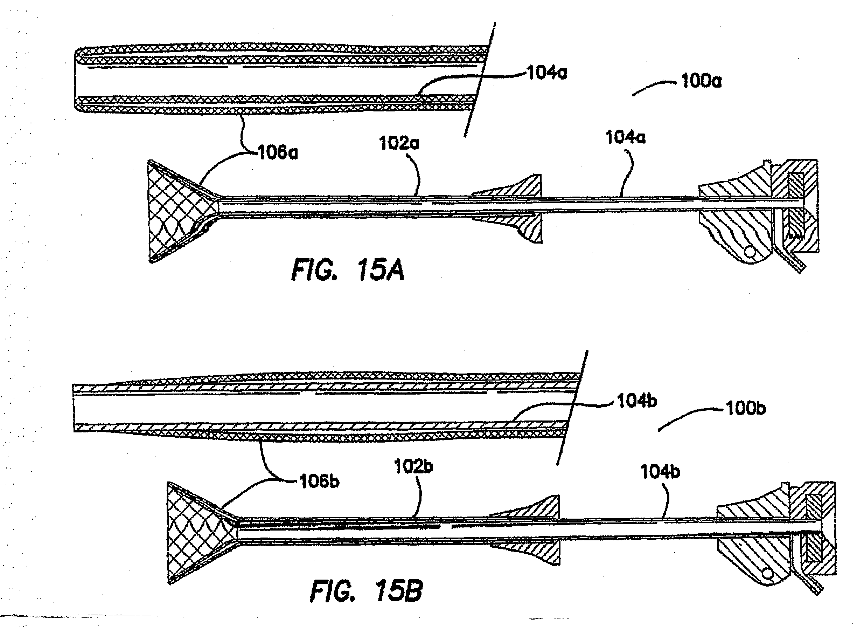

Referring to FIG. 15(a), there is shown a side sectional view of an access device 100a in accordance with another aspect of the invention having a first outer flexible tube 102a, a second inner, coaxial, flexible tube 104a, and an expandable portion 106a disposed at a distal portion of the first and second tubes 102, 104. With this aspect, the expandable portion 106a is formed with a braid material and both the first outer tube 102a and the second inner tube 104a are also formed with a braid material or are braid-reinforced. FIG. 15(b) illustrates an access device 100b in accordance with another aspect of the invention having an expandable portion 106b being formed with a braid material and at least one of a first outer tube 102b and a second inner tube 104b being formed with a braid material or braid-reinforced.

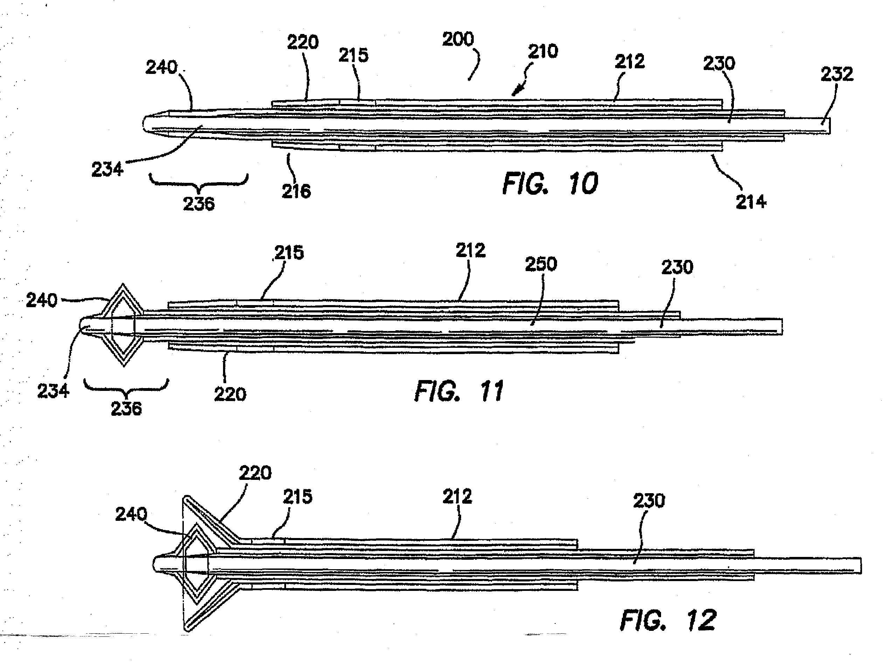

Referring to FIG. 10, there is shown an access device 200 in accordance with another aspect of the invention having a sheath assembly 210 and an obturator assembly 230 inserted through the sheath assembly 210. The sheath assembly 210 comprises an outer tube 212 having a proximal tube end 214 and a distal tube end 216. The sheath assembly 210 further comprises an expandable containment member 220, which is connected to the outer tube 212 near the distal tube end 216. The obturator assembly 230 comprises a proximal obturator end 232 and a distal obturator end 234. The obturator end area 236 includes an obturator expandable portion or cone 240 and a proximal portion. The expandable containment member 220 of the sheath assembly 210 is operably connected to the proximal portion of the obturator end area 236. An obturator handle of the obturator assembly 230 is connected to an accessory device of the sheath assembly 210 via a handle connector.

The outer tube 212 of the sheath assembly 210 is connected to the expandable containment member 220 by a connector portion 215. More specifically, the connector portion 215 is located between the expandable containment member 220 and the outer tube 212 and comprises a braid-reinforced portion or a semi-rigid portion of solid walled tubing. The expand able containment member 220 may comprise a braided tubular component, and the outer tube 212 may comprise thin-walled reinforced tubing as discussed above (e.g., wire-reinforced tubing or individual ring-shaped elements forming a tubular structure). The expandable containment member 220 is joined to the connector portion 215 by either bonding or fusion. Similarly, the connector portion 215 is joined to the outer tube 212 by either bonding or thermal fusion. The connector portion 215 may further comprise a solid plastic component and operates to removably connect the outer tube 212 to a tube connector of an accessory device such as the obturator assembly 230. With this aspect, the connector portion 215 may comprise threads which fit into the tube connector for a snug fit.

A lumen is formed within the outer tube 212 between the distal tube end 216 and the proximal tube end 214. This lumen is preferably sized and configured to accommodate a shaft portion of the obturator assembly 230. The lumen of the outer tube 212 may also removably accommodate other instruments.

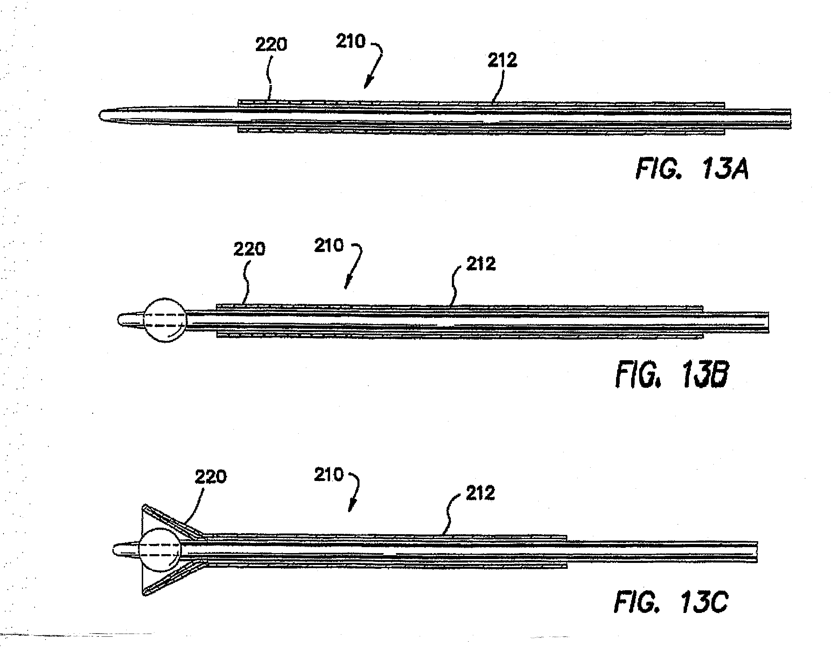

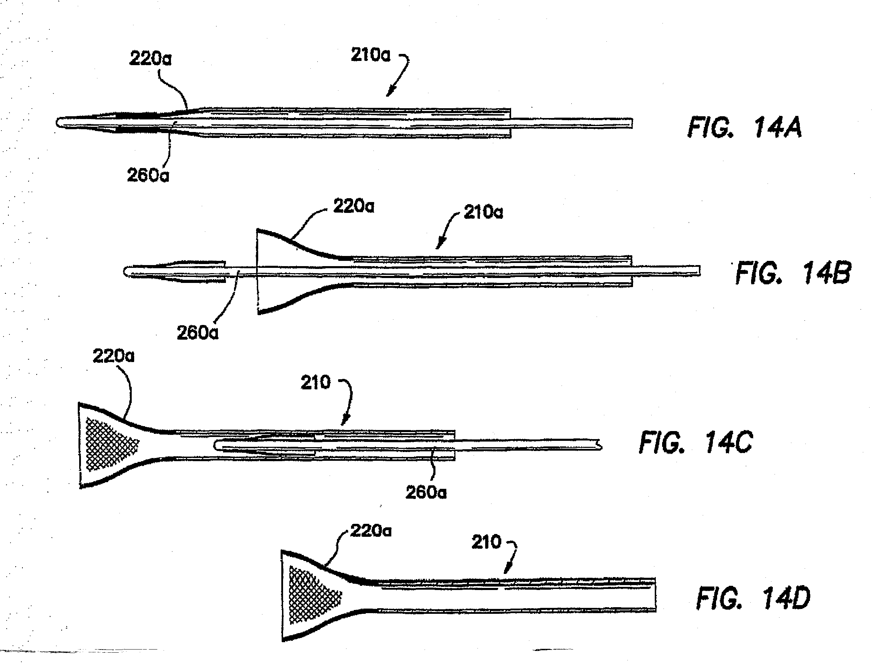

Referring to FIG. 11, the obturator assembly 230 comprises an obturator shaft 250 having an expandable cone 240, an obturator end area 236, and an obturator handle (not shown). The expandable cone 240 can be radially expanded and contracted by movement of the obturator handle. According to the invention, a distal obturator end 234 of the obturator assembly 230 is inserted through a handle connector of the sheath assembly 210. The distal obturator end 234 is then moved through the lumen of the outer tube 212, and out of the expandable containment member 220 of the sheath assembly 210. The obturator assembly 230 may further include threads to securely connect with the sheath assembly 210. Once the sheath assembly 210 and the obturator assembly 230 are attached, the obturator end area 236 extends distally out of the expandable containment member 220 of the sheath assembly 210. With this embodiment, the expandable containment member 220 can be expanded by movement between the handle connector of the sheath assembly 210 and the obturator handle of the obturator assembly 230. This movement corresponds to relative movement of the outer flexible tube 212 and the obturator assembly 230 in opposite directions. After the access device 200 is placed into a body passage and the containment member 220 is expanded, the obturator assembly 230 can then be removed from the access device, to thereby provide an unobstructed lumen within the outer tube 212. The lumen of the outer tube 212 can then facilitate insertion and removal of instruments and materials. For example, a therapeutic balloon catheter can be inserted into the lumen to facilitate removal of embolus or thrombus as illustrated in FIGS. 13(a) - 13(c). The access device 200 of the invention may further include a guidewire, which is adapted to be inserted through the obturator assembly 230. The guidewire acts as a stiffener and as a leader for the access device. FIGS. 14(a) -14(d) illustrate a cowling 260a that operates to cover an expandable containment member 220a of a sheath assembly 210a in another aspect of the invention.

It will be understood that many other modifications can be made to the various disclosed embodiments without departing from the spirit and scope of the invention. For these reasons, the above description should not be construed as limiting the invention, but should be interpreted as merely exemplary of the disclosed embodiments.

## Claims

1. An access device adapted for use in a body conduit, comprising:
an outer tube having a proximal tube end, a distal tube end, and a lumen extending between the proximal tube end and the distal tube end;
an obturator assembly having a proximal obturator end a distal obturator end, and being removably and concentrically disposed within the lumen of the outer lube;
an expandable portion having a proximal end being connected to the distal tube end and a distal end being detachably connected to the distal obturator end, the expandable portion being expandable by relative movement between the expandable portion proximal end and the expandable portion distal end, resulting from relative movement of the outer tube and the obturator assembly in opposite directions; and
the outer tube comprises a wire-reinforced tube or is formed from a plurality of individual, discrete, generally ring-shaped elements arranged in series and fused or bonded together forming a continuous tubular structure.

2. The access device of claim 1, wherein the expandable portion in the high-profile state has the shape of a cone or a funnel.

3. The access device of claim 1, wherein the expandable portion includes a woven mesh with interstices.

4. The access device of claim 2, wherein the expandable portion is formed with a braid material.

5. The access device of claim 1, wherein the ring-shaped elements are formed of a thermoplastic or a thermoset material.,

6. The access device of claim 1, wherein the ring-shaped elements include at least one of plastic rings, metallic rings, un-reinforced plastic rings and metal reinforced plastic rings assembled along the length of the outer tube to provide variable flexibility and kink-resistance.

7. The access device of claim 1, wherein the ring-shaped elements have different flexural modulus.

8. The access device of claim 1, wherein the ring-shaped elements include a combination of flexible and rigid ring-shaped elements assembled along different portions or sections of the outer tube.

9. The access device of claim 8, wherein as the outer tube is bent, twisted or curved, the rigid ring-shaped elements provide reinforcement to maintain the size and shape of the lumen and the flexible ring-shaped elements operate to stretch and compress to prevent kinking..

10. The access device of claim 1, wherein the ring-shaped elements are metallic and are bonded with a resilient, flexible elastomeric adhesive.

11. The access device of claim 10, wherein the ring-shaped elements have different lengths and are fused closer or further apart to one another depending on the characteristics of a portion or section of the outer tube.

12. The access device of claim 1, wherein the wire-reinforced tube is manufactured by the process comprising the steps of:
coating a wire with a plastic material;
wrapping the coated wire around a mandrel forming a plurality of windings; and
heating the wound coated wire until the plastic material melts and bonds the windings forming the wire-reinforced tube.

13. The access device of claim 12, wherein the plastic material comprises at least one of polyurethane, a thermoplastic material and a thermoset material.

14. The access device of claim 13, wherein the wire comprises at least one of a metallic material and a second plastic material.

15. The access device of claim 13, wherein the wire is coated with the plastic material in a coextrusion process.
